**Europäisches Patentamt**

**European Patent Office**

**Office européen des brevets**

(11) Veröffentlichungsnummer: **0 203 555**
A2

(12) # EUROPÄISCHE PATENTANMELDUNG

(21) Anmeldenummer: **86107093.6**

(22) Anmeldetag: **24.05.86**

(51) Int. Cl.⁴: **A 61 K 37/24,** A 61 K 37/32, A 61 K 37/36, A 61 K 37/38, A 61 K 37/40

(30) Priorität: **31.05.85 DE 3519579**

(43) Veröffentlichungstag der Anmeldung: **03.12.86** **Patentblatt 86/49**

(84) Benannte Vertragsstaaten: **AT BE CH DE FR GB IT LI LU NL SE**

(71) Anmelder: **Bissendorf Peptide GmbH, Burgwedeler Strasse 25, D-3002 Wedemark 2 (DE)**

(72) Erfinder: **Wagner, Thomas O.F., Dr.,** **Rosskampstrasse 25, D-3000 Hannover 81 (DE)**

(74) Vertreter: **Werner, Hans-Karsten, Dr. et al, Deichmannhaus am Hauptbahnhof, D-5000 Köln 1 (DE)**

(54) Mittel zur Funktionsdiagnose der Hypophyse und zur Therapie von Störungen der Hypophysenfunktionen.

㊼ Mittel zur Funktionsdiagnose der Hypophyse und zur Therapie von Störungen der Hypophysenfunktionen, enthalten mindestens zwei der vier Hormone GHRH (zur Stimulation von Wachstumshormon), CRF (zur Stimulation von Corticotropin), TRH (zur Stimulation von Schilddrüsen stimulierendem Hormon und Prolaktin) und LHRH (zur Stimulation von luteinisierendem Hormon und Follikel stimulierendem Hormon) in wirksamen Mengen in steril ampullierter, injizierbarer Form. Die Hormone sind miteinander verträglich und lagerstabil. Sie führen bei gleichzeitiger Applikation zu keinen störenden Interferenzen.

## Mittel zur Funktionsdiagnose der Hypophyse und zur Therapie von Störungen der Hypophysenfunktionen

Die vorliegende Erfindung betrifft Mittel zur Funktionsdiagnose der Hypophyse und zur Therapie von Störungen der Hypophysenfunktionen.

Die Hypophyse ist eine der vielseitigsten Drüsen, die regulierend in die verschiedensten Bereiche der Körperfunktionen eingreift. Die Überprüfung der normalen oder gestörten Funktion des Hypophysenvorderlappens erfordert bisher eine aufwendige und für den Patienten nicht ungefährliche Diagnostik. Diese beinhaltet in der Regel:

1. die Untersuchung der thyreotropen (Schilddrüsenachse) und lactotropen (Prolactinachse) Funktion mit Hilfe von TRH (Thyreotropin Releasing Hormon),

2. die Untersuchung der gonadotropen Partialfunktion mit Gonadotropin Releasing Hormon (GnRH oder LHRH), und

3. die Durchführung eines Insulinhypoglycaemie-Testes. Dieser Test macht sich die Tatsache zunutze, daß eine Unterzuckerung (Hypoglycaemie) einen erheblichen Stress darstellt, der zu einer Aktivierung der corticotropen (Nebennierenachse) und somatotropen (Wachstumsachse) Funktionen des Hypophysenvorderlappens führt.

Diese Unterzuckerung, die durch Gabe von Insulin (als intravenöse Bolusinjektion) ausgelöst wird, hat neben der unangenehmen subjektiven Empfindung für den untersuchten Patienten (Herzrasen, Durst- und Heißhungergefühle, Schweißausbruch, innere Unruhe, Angst etc.) auch die große Gefahr eines Unterzuckerungsschocks.

Neuere Untersuchungen haben gezeigt, daß die Überprüfung der beiden Achsen Nebenniere und Wachstum vorzugsweise durch die spezifischen Releasing Hormone CRF (zur Stimulation von Corticotropin) und GHRH (zur Stimulation von Wachstumshormon) erfolgen kann. Es hat sich weiter gezeigt, daß es prinzipiell möglich ist, mit Hilfe der vier Hormone TRH, LHRH, CRF und GHRH alle hypophysären Partialfunktionen ohne das Risiko einer Unterzuckerung überprüft werden können.

Ein wesentlicher Nachteil der bisherigen Untersuchungstechnik ist die Verknüpfung verschiedener Prinzipien: Während TRH und LHRH eine direkte Stimulation der Hypophyse bewirken und somit auch eine direkte Aussage über die Hypophysenfunktion zuläßt, stellt die Insulinhypoglycaemie eine indirekte Stimulation dar. Über die Unterzuckerung kommt es zur hypothalamischen Aktivierung; zur Ausschüttung der körpereigenen Releasing Hormone GHRH und CRF und erst über diese hypothalamische Aktivierung zur Stimulation der Hypophyse. Somit ist dieser Test auch gestört bei intakter Hypophysenfunktion, aber gestörter hypothalamischer Sekretion. Durch die Kombination von TRH und LHRH mit GHRH und CRF sind alle Hypophysenfunktionen direkt überprüfbar, und falsch negative Ergebnisse durch Defekte im Hypothalamus können ausgeschlossen werden.

Ein weiterer Nachteil der bisher durchgeführten Hypophysenfunktionsteste bestand darin, daß Mischungen von Insulin mit anderen Hormonen nur eine sehr geringe Haltbarkeit aufweisen und daher nicht zu vorgefertigten, haltbaren Kombinationen verarbeitet werden können.

Da bekannt war, daß bei lang andauernder und hochdosierter Behandlung mit Glukokortikoiden die Ausschüttung von TSH nach Injektion von TRH unterdrückt wird, mußte befürchtet werden, daß auch bei gleichzeitiger Applikation von TRH und CRF unerwünschte Interferenzen auftreten und daher diese Teste nur mit ausreichendem zeitlichen Abstand nacheinander durchgeführt werden sollten.

Die Erfindung hat sich die Aufgabe gestellt, die Funktionsdiagnose der Hypophyse sicherer und einfacher durchführbar zu machen und dabei möglichst schnell zu zuverlässigen Ergebnissen zu kommen. Es wurde jetzt gefunden, daß es ohne weiteres möglich ist, die vier Hormone GHRH, CRF, TRH und LHRH praktisch gleichzeitig zu applizieren und dabei die Stimulierung der Ausschüttung von Wachstumshormon, Corticotropin, Schilddrüsen stimulierendem Hormon und Prolaktin und luteinisierendem Hormon und Follikel stimulierendem Hormon nebeneinander zu bestimmen. Weiterhin wurde festgestellt, daß diese vier Hormone in lyophilisierter Form miteinander verträglich sind und deshalb steril ampulliert in injizierbarer Form kombiniert werden können. Für einen vollständigen Funktionstest der Hypophyse ist es daher möglich, alle vier Hormone in wirksamen Mengen in steril ampullierter, injizierbarer Form miteinander zu kombinieren und zu applizieren. Für etwaige differentialdiagnostische Untersuchungen können gewünschtenfalls auch ein oder zwei der vier Hormone weggelassen werden.

Weiterhin wurde festgestellt, daß die Verträglichkeit der Hormone in kombinierten Injektionslösungen es ermöglicht, diese Kombinationen in der Therapie von Störungen von Hypophysenfunktionen einzusetzen. Von besonderem Interesse ist die Therapie von Pubertäts- und Wachstumsstörungen. Hierzu wird eine Kombination von LHRH und GHRH verwendet.

Gegenstand der vorliegenden Erfindung sind somit Mittel zur Funktionsdiagnose der Hypophyse und zur Therapie von Störungen der Hypophysenfunktionen, enthaltend mindestens zwei der vier Hormone GHRH (zur Stimulation von Wachstumshormon), CRF (zur Stimulation von Corticotropin), TRH (zur Stimulation von Schilddrüsen stimulierendem Hormon und Prolaktin) und LHRH (zur Stimulation von luteinisierendem Hormon und Follikel stimulierendem Hormon) in wirksamen Mengen in steril ampullierter, injizierbarer Form. Ein weiterer Gegenstand der Erfindung ist die Verbindung von mindestens zwei der vier Hormone GHRH, CRF, TRH und LHRH in wirksamen Mengen in steril ampullierter, injizierbarer Form zur Funktionsdiagnose der Hypophyse.

Schließlich betrifft die Erfindung die Verwendung von mindestens zwei der vier Hormone GHRH, CRF, TRH und LHRH zur Herstellung von Mitteln zur Funktionsdiagnose der Hypophyse und zur Therapie von Störungen der Hypophysenfunktionen.

Die wirksamen Mengen der vier Hormone sind in Kombination die gleichen wie bei der Applikation als Einzelsubstanz. Sie hängen somit insbesondere auch vom Körpergewicht der zu untersuchenden Patienten ab. Bei GHRH kommen prinzipiell die verschiedenen Faktoren mit Moleküllängen von 1 - 29, 1 - 40 und 1 - 44 in Frage, die aus humanen pankreatischen Tumoren isoliert und anschließend synthetisiert wurden. Ins-

- 5 -

0 203 555

besondere kommt synthetisches GHRH/1-44 in Frage, welches die Wachstumshormonausschüttung aus der Hypophyse stimuliert und mit dem hypothalamischen GHRH identisch ist. Das Produkt wird beispielsweise von der Anmelderin unter der Bezeichnung GHRH-Bissendorf vertrieben. Vom GHRH (1 - 44) kommen 50 - 100 µm zur Anwendung.

Das Hormon CRF (Corticotropin Releasing Factor) ist ein Peptid mit 41 Aminosäuren, welches ebenfalls vollsynthetisch herstellbar ist und von der Anmelderin unter der Bezeichnung CRF-Bissendorf vertrieben wird. Auch dieses Hormon wird meist in Mengen von ca. 100 µg pro Dosis eingesetzt.

Das Hormon TRH wird in Mengen von ca. 200 bis 400 µg eingesetzt. Dieses Hormon, welches auch Protirelin genannt wird, ist beispielsweise unter der Bezeichnung Relefact $^R$ TRH 200 (Hoechst AG) zugänglich.

Das Hormon LHRH, welches auch unter dem Namen Gonadorelin bekannt ist, wird ebenfalls von der Hoechst AG angeboten unter der Bezeichnung Relefact $^R$ LH-RH in Dosierungen von 0,025 und 0,1 mg.

Der Wirkungseintritt aller vier Hormone kann bereits wenige Minuten nach der Applikation festgestellt werden und gewünschtenfalls messend verfolgt werden. Im allgemeinen genügen aber Probenentnahmen kurz vor der Applikation sowie 15, 30, 45 und 60 Minuten nach der Injektion der Hormonkombination. Etwa zwei Stunden nach der Injektion werden meist schon wieder die Ausgangswerte beobachtet bzw. die Werte, die dem Tagesrhythmusspiegel entsprechen.

Bei Fehlverhalten der Hypophyse wird das eine oder andere hypophysere Hormon nach Stimulation mit dem entsprechenden Releasing Hormon nicht ausgeschüttet. Bei Anwendung des erfindungsgemäßen kombinierten Hypophysen-Testes mit allen Releasing Hormonen schüttet eine intakte Hypophyse alle der o.a. Hormone aus. Bei Störungen der Hypophysenfunktionen läßt sich diese Störung durch die nach dem Test durchgeführte Messung aller Hypophysenhormone im Blut genau lokalisieren. Wird beispielsweise eines der Hormone nicht ausgeschüttet, ist die Störung der Hypophysenfunktion auf diese Zellgruppe beschränkt, die das betreffende Hormon ausschütten sollte. Wird keines der o.a. Hormone ausgeschüttet, sind alle Zellgruppen von der Störung betroffen.

Es wurden folgende vergleichende Untersuchungen durchgeführt:

An vier aufeinander folgenden Tagen wurden die vier Hormone nacheinander appliziert und die Ausschüttung der entsprechenden Hypophysenhormone ACTH, Cortisol, STH, TSH, LH, FSH und PRL messend verfolgt. Weiterhin wurden die vier Hormone gleichzeitig appliziert. Die Meßergebnisse waren völlig vergleichbar und zeigten, daß keine Interferenzen vorliegen.

Es wurde weiterhin festgestellt, daß die vier Hormone in lyophilisierter miteinander verträglich sind im Gegensatz zu Mischungen mit Insulin, welche nur eine sehr geringe Haltbarkeit aufwiesen. Es ist daher möglich, alle gewünschten Zweier- und Dreierkombinationen sowie die Viererkombination herzustellen und steril ampulliert in injizierbarer Form zur Verfügung zu stellen. Zur Therapie von Pubertäts- und Wachstumsstörungen hat sich weiterhin die Kombination aus LHRH und GHRH besonders bewährt. Es ist anzunehmen, daß nach intensiverer Durchführung von Funktionsdiagnosen der Hypophyse in Zukunft auch häufiger Therapien von Störungen der Hypophysenfunktionen zur Anwendung kommen. Hierbei wird es ebenfalls möglich sein, alle gewünschten Zweier- oder Dreierkombinationen, gewünschtenfalls sogar die Viererkombination als Therapeutikum zur Verfügung zu stellen.

P a t e n t a n s p r ü c h e

1. Mittel zur Funktionsdiagnose der Hypophyse und zur Therapie von Störungen der Hypophysenfunktionen, enthaltend mindestens zwei der vier Hormone GHRH (zur Stimulation von Wachstumshormon), CRF (zur Stimulation von Corticotropin), TRH (zur Stimulation von Schilddrüsen stimulierendem Hormon und Prolaktin) und LHRH (zur Stimulation von luteinisierendem Hormon und Follikel stimulierendem Hormon) in wirksamen Mengen in steril ampullierter, injizierbarer Form.

2. Mittel gemäß anspruch 1, dadurch gekennzeichent, daß es LHRH und GHRH zur Therapie von Pubertäts- und Wachstumsstörungen enthält.

3. Mittel gemäß Anspruch 1, dadurch gekennzeichnet, daß es alle vier Hormone zur Funktionsdiagnose der Hypophyse enthält.

4. Verwendung von mindestens zwei der vier Hormone GHRH, CRF, TRH und LHRH in wirksamen Mengen in steril ampullierter, injizierbarer Form zur Funktionsdiagnose der Hypophyse.

5. Verwendung von mindestens zwei der vier Hormone GHRH, CRF, TRH und LHRH zur Herstellung von Mitteln zur Funktionsdiagnose der Hypophyse und zur Therapie von Störungen der Hypophysenfunktionen.